# EUROPÄISCHE PATENTSCHRIFT

(11) **EP 0 608 725 B1**
(45) Veröffentlichungstag und Bekanntmachung des Hinweises auf die Patenterteilung: **17.05.2000**
(21) Anmeldenummer: 94100501.9
(22) Anmeldetag: 14.01.1994
(51) Int. Cl.: C07C 209/68, C07C 229/30, C07C 255/30, C07C 225/14, C07C 211/29

(54) **Verfahren zur Herstellung von Aminomethylenverbindungen**
Process for preparing aminomethylene compounds
Procédé pour la préparation de composés aminométhyléniques

(30) Priorität: 27.01.1993 DE 4302156
(43) Veröffentlichungstag der Anmeldung: 03.08.1994
(73) Patentinhaber: BAYER AG, 51368 Leverkusen (DE)
(72) Erfinder: Kraus, Helmut, Dr., D-51519 Odenthal (DE); Müller, Nikolaus, Dr., D-40789 Monheim (DE); Marzolph, Gerhard, Dr., D-51061 Köln (DE); Beitzke, Bernhard, Dr., D-51503 Rösrath (DE)

(56) Entgegenhaltungen:
- DE-A- 3 925 720
- GB-A- 917 436
- ORGANIC SYNTHESES, Band 63, 1985 A.D. BATCHO et al. "Indoles from 2-methylnitrobenzenes by condensation with formamide acetals followed by reduction: 4-benzyloxyindole" Seiten 214-226
- JOURNAL OF HETEROCYCLIC CHEMISTRY, Band 24, Nr. 1, January - February 1987 J.R. BECK et al. "Synthesis of 1-Aryl-5-(trifluoro- methyl)-1H-pyrazole-4-car- boxylic Acids and Esters" Seiten 739-740
- LIEBIGS ANNALEN DER CHEMIE, 1980 W. KANTLEHNER et al. "Um- setzungen von tert-Butoxy-N, N,N',N'-tetramethylmethan- diamin mit NH- und CH-aciden Verbindungen" Seiten 344-357

## Beschreibung

Die Erfindung betrifft ein Verfahren zur Herstellung von Aminomethylenverbindungen (Enaminen) durch Umsetzung von C-H-aciden Verbindungen mit Formamidacetalen unter Zusatz einer katalytischen Menge eines sekundären Amins.

Enamine, wie beispielsweise aminomethylenierte Malonester, Essigester, Nitrile, 2-Nitrotoluole oder Picoline sind wichtige Bausteine zur Herstellung von Heterocyclen, wie Pyrazolen, Pyrimidinen, Chinolinen oder Indolen, die als pharmazeutische Wirkstoffe oder Pflanzenschutzmittel Verwendung finden.

In der Vergangenheit wurde eine Vielzahl von Verfahren zur Aminomethylenierung beschrieben; einige wichtige von ihnen sind in DE-OS 39 25 720 erwähnt und diskutiert.

Die Verwendung von Orthoamiden führt zu technisch einfachen Verfahren, da neben dem gewünschten Reaktionsprodukt nur leichtflüchtige Komponenten entstehen. Je nach C-H-Acidität des Ausgangsmaterials reicht das technisch verfügbare DMF-Acetal aus oder man muß Aminalester oder sogar Tris-aminomethane verwenden.

In J. Org. Chem. 46 (1981), 1752 ist beschrieben, daß die Reaktivität des DMF-Acetals drastisch gesteigert werden kann, wenn man bei der Umsetzung mindestens ein Äquivalent eines cyclischen sekundären Amins zusetzt. Pyrrolidin als reaktives Amin senkt beispielsweise die Reaktionszeit einer Aminomethylenierung von 51 auf 3 Stunden (Org. Synth. 63 (1985), 214).

Die erhöhte Reaktivität wird mit der intermediären Bildung von Pyrrolidino-aminalester bzw. -tris-amino-Verbindungen erklärt. Solche Spezies dimerisieren jedoch bei relativ niedrigen Temperaturen zu Tetraaminoethenen, was zu drastischen Ausbeuteminderungen führen kann. So reagiert Tri-pyrrolidinomethan schon ab 100°C zu Tetra-pyrrolidino-ethen, während Tris(dimethylamino)-methan erst oberhalb von 160°C dimerisiert.

Im gebräuchlichen Reaktionsmedium DMF läuft als weitere Nebenreaktion die Formylierung der cyclischen Amine ab. Bei der Aminomethylenierung mit DMF-Acetal unter Zusatz cyclischer Amine erhält man immer ein Gemisch der Enamine, meist überwiegt die cyclische Aminoverbindung neben 10 bis 30 % an Dimethylamino-Produkt. Dies bedeutet, daß etwa 1 Äquivalent des teuren cyclischen Amins verbraucht wird und außerdem die gleiche Menge an Dimethylamin freigesetzt wird, welches aufwendig entsorgt werden muß.

Überraschend wurde festgestellt, daß es möglich ist, die Reaktivität von Dialkylamino-Formamid-Acetalen beträchtlich zu erhöhen, wenn man eine nicht stöchiometrische Menge eines nicht cyclischen sekundären Amins zusetzt.

Die Erfindung betrifft demnach ein Verfahren zur Herstellung von Aminomethylenverbindungen der Formel 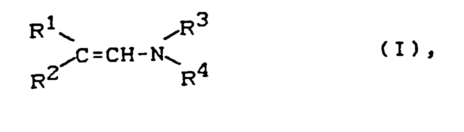 in der
- R¹: Nitro, Cyano, Isonitrilo, COR⁹, CSR⁹, COOR⁹, COSR⁹, CON(R⁹,R¹⁰), SO₂R⁹, SO₃R⁹, CONH₂ oder einen an der Position des bevorzugten nukleophilen Angriffs elektronenarmen Aromaten oder Heteroaromaten darstellt,
- R²: den Bedeutungsumfang von R¹, jedoch unabhängig von R¹, annehmen kann und außerdem Wasserstoff, OR⁹, SR⁹, N(R⁹,R¹⁰), geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatom 1 oder 2 aus der Gruppe von H, O, S sind, darstellt,
- R³ und R⁴: unabhängig voneinander geradkettiges oder verzweigtes C₁-C₈-Alkyl, C₂-C₈-Alkenyl, C₂-C₈-Alkoxyalkyl oder C₃-C₈-Alkoxyalkenyl darstellen und
- R⁹ und R¹⁰: unabhängig voneinander geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatom 1 oder 2 aus der Gruppe von N, O, S sind, darstellen, wobei weiterhin R⁹ und R¹⁰ gemeinsam mit dem N-Atom, das sie substituieren, einen 5- bis 8-gliedrigen Ring bilden können, der ein weiteres Heteroatom aus der Gruppe von N, O, S enthalten kann,
das dadurch gekennzeichnet ist, daß man CH-acide Verbindungen der Formel 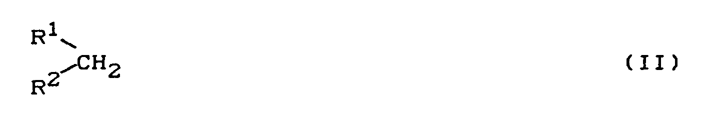 mit Formamidacetalen der Formel 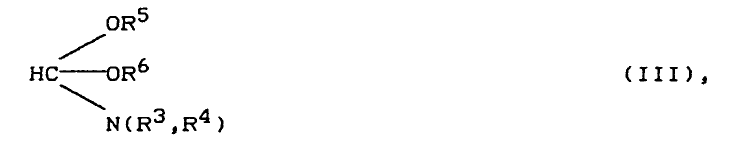 in denen
R¹ bis R⁴ den genannten Bedeutungsumfang haben und
R⁵ und R⁶ unabhängig von R³ und R⁴ und unabhängig voneinander den für R³ und R⁴ gegebenen Bedeutungsumfang annehmen,
in Gegenwart eines sekundären Amins der Formel 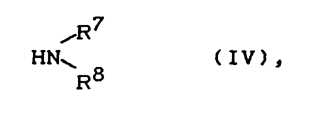 in der
R⁷ und R⁸ unabhängig von R³ und R⁴ und unabhängig voneinander den für R³ und R⁴ gegebenen Bedeutungsumfang annehmen,
oder in Gegenwart von Substraten, die solche Amine unter den Reaktionsbedingungen freisetzen, bei 0 bis 200°C mit oder ohne Lösungsmittel umsetzt, wobei das molare Verhältnis von C-H-acider Verbindung zu Formamidacetal 1:3 bis 10:1 beträgt und die Menge an sekundärem Amin 1 bis 500 Mol-%, bezogen auf das Formamidacetal, beträgt.

Geradkettiges oder verzweigtes C₁-C₈-Alkyl ist beispielsweise Methyl, Ethyl, Propyl, Isopropyl, Butyl, Isobutyl, tert.-Butyl, die isomeren Pentyle, Hexyle, Octyle, bevorzugt die genannten C₁-C₄-Alkylreste.

Geradkettiges oder verzweigtes C₂-C₈-Alkenyl ist beispielsweise Vinyl, Propenyl, Allyl, die isomeren Butenyle, Pentenyle, Hexenyle oder Octenyle, bevorzugt die genannten C₃-C₄-Alkenylreste.

Geradkettiges oder verzweigtes C₂-C₈-Alkoxyalkyl ist beispielsweise Methoxymethyl, Ethoxymethyl sowie weitere Reste aus der Gruppe C₃-C₉-Alkyl, in welchem eine CH₂-Gruppe durch ein O-Atom ersetzt ist.

Geradkettiges oder verzweigtes C₃-C₈-Alkoxyalkenyl ist beispielsweise Methoxyvinyl, Ethoxyvinyl, Methoxyallyl, 2-Methoxy-propenyl und andere aus der Gruppe von C₄-C₉-Alkenyl, worin eine CH₂-Gruppe durch ein O-Atom ersetzt ist.

C₃-C₈-Cycloalkyl ist beispielsweise Cyclopropyl, Methylcyclopropyl, Dimethyl-cyclopropyl, Cyclobutyl, Methylcyclobutyl, Cyclopentyl, Methyl-cyclopentyl, Cyclohexyl, Methyl-cyclohexyl, Dimethyl-cyclohexyl, Cycloheptyl, Cyclooctyl, bevorzugt Cyclopropyl, Cyclopentyl und Cyclohexyl, sowie deren Methyl- oder Dimethyl-Derivate.

C₆-C₁₂-Aryl ist beispielsweise Phenyl, Naphthyl oder Biphenylyl, bevorzugt Phenyl.

C₇-C₁₀-Aralkyl ist beispielsweise Benzyl, 1-Phenylethyl, 2-Phenylethyl und weitere dem Fachmann bekannte Reste dieser Art, bevorzugt Benzyl.

Als carbocyclische oder heterocyclische Aromatenreste kommen solche in Frage, deren Verknüpfungsstelle mit dem C-Atom in Formel (II) bzw. der entsprechenden Stelle in Formel (I) diejenige ist, die wegen der dortigen relativen Elektronenarmut die bevorzugte Position für einen nukleophilen Angriff ist. Diese bevorzugte Position ist dem Fachmann geläufig. Ein wichtiges Beispiel ist etwa die ortho- oder die para-Position beim Nitrobenzol. Wichtige Aromatenreste dieser Art im Rahmen von R¹ sind beispielsweise 1-Imidazolyl, 3-Imidazolyl, 2-Pyrimidyl, 4-Pyrimidyl, 2-Nitrophenyl, 4-Nitrophenyl, 2,3-Dinitrophenyl, 2,4-Dinitrophenyl, 2-Cyanophenyl, 2,4-Dichlorphenyl, 2-Pyridyl, 4-Pyridyl, 2-Chinolyl, 2-Carboxyphenyl.

Als 5- bis 8-gliedriger gesättigter oder ungesättigter heterocyclischer Ring, dessen Heteroatome 1 oder 2 aus der Gruppe von N, O und S sind, seien genannt: Pyrrol, Furan, Thiophen, Pyrrolidin, Pyrrolin, Pyrazol, Pyrazolidin, Imidazol, Imidazolidin, Thiazol, Thiazolidin, Oxazol, Pyridin, Pyrimidin, Piperazin, das am N-Atom durch C₁-C₄-Alkyl oder durch Hydroxy-C₁-C₄-alkyl substituiert sein kann, Morpholin, Dioxan, Dioxolan, Pyran, Azepin, Azocin, Dihydroazocin, Isoxazol, Dioxolan, Dioxazol, Isothiazol, Pyridazin und Pyrazin. Es ist dem Fachmann bekannt, daß ungesättigte heterocyclische Ringe einen mehr oder weniger stark ausgeprägten aromatischen Charakter haben können. In bevorzugter Weise seien die nicht aromatischen Ringe Morpholin, Pyrrolidin und Piperidin, das durch C₁-C₄-Alkyl oder durch Hydroxy-C₁-C₄-alkyl substituiert sein kann, genannt.

Formamidacetale können beispielsweise durch Umsetzung von Methyleniminiumsalzen mit Alkoholaten hergestellt werden (Chem. Ber. 97, 3397, (1968)).

Die Reaktion des erfindungsgemäßen Verfahrens kann beispielhaft wie folgt dargestellt werden:

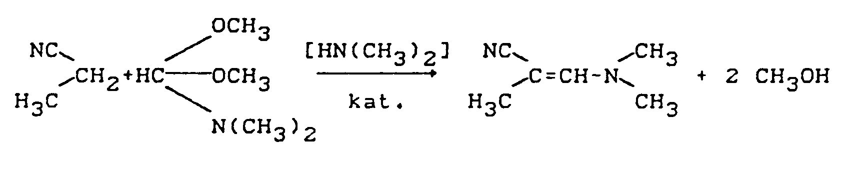

Die Umsetzung des erfindungsgemäßen Verfahrens kann ohne weiteres Lösungs- oder Verdünnungsmittel durchgeführt werden, wenn die Reaktionspartner flüssig sind. Sie kann ferner im Überschuß eines der beiden Reaktionspartner durchgeführt werden, wenn dieser überschüssige Reaktionspartner flüssig ist. Es kann ferner ein Lösungs- oder Verdünnungsmittel angewandt werden.

Als Lösungsmittel können solche eingesetzt werden, deren Acidität geringer ist als die der C-H-aciden Verbindung und die unter den Reaktionsbedingungen keine oder nur geringfügige Austausch- oder Additionsreaktionen mit dem sekundären Amin eingehen. Geeignete Lösungsmittel gehören der Gruppe der Alkohole, Kohlenwasserstoffe, halogenierten Kohlenwasserstoffe, tertiären Amine, Ketone, Amide, insbesondere Dialkylamide, Nitrile, Ether, Ester, Phosphorsäureamide, insbesondere der Peralkyl-phosphoramide, Phosphorsäureester, Sulfone, Sulfolane, N-Alkyllactame, Kohlensäureester und Harnstoffderivate, insbesondere Peralkyl-harnstoffderivate. Von den genannten Gruppen sind die aprotisch polaren Lösungsmittel und die Alkohole bevorzugt. Beispiele für Lösungsmittel aus den genannten Gruppen sind:

Petrolether, Toluol, Xylol, Chlortoluol, Ligroin, 1,2-Dichlorethan, Triethylamin, Aceton, Acetonitril, Essigsäure-tert.-butylester, Dimethylcarbonat, Dimethylacetamid, Dimethylformamid (DMF), N-Methyl-pyrrolidon (NMP), N-Methyl-caprolactam (NMC), Tetramethylharnstoff, Dimethylsulfoxid, Diethylsulfon, Methyl-tert.-butylether (MTBE), Tetrahydrofuran (THF), Methanol und Isopropanol, bevorzugt Aceton, Acetonitril, Essigsäure-tert.-butylester, Dimethylcarbonat, Dimethylacetamid, DMF, NMP, NMC, Tetramethylharnstoff, Dimethylsulfoxid, Diethylsulfon, MTBE, THF, Methanol und Isopropanol.

Die Reaktionstemperatur liegt bei 0 bis 200°C, bevorzugt bei 20 bis 170°C. Die Reaktionszeit ist von der Größe des Reaktionsansatzes und von der C-H-Acidität des Ausgangsmaterials sowie von der Temperatur abhängig und liegt im allgemeinen zwischen 0,5 und 36 Stunden.

Das Verhältnis von C-H-acider Verbindung zu Formamidacetal beträgt 1:3 bis 10:1, bevorzugt 1:2 bis 5:1. Der Anteil an sekundärem Amin ist unabhängig von der Stöchiometrie der chemischen Reaktion im erfindungsgemäßen Verfahren, wie sie oben beispielhaft formuliert wurde, und kann 1 bis 500 Mol-%, bezogen auf das Formamidacetal, bevorzugt 2 bis 300 Mol-% und besonders bevorzugt im unterstöchiometrischen Bereich von 2 bis 25 Mol-% betragen. Insbesondere die unterstöchiometrische Menge vereinfacht die Reaktionsführung (geringer Druck des Reaktionssystems) und die Entsorgung des Amins (Aufarbeitung, Recyclisierung oder Verbrennung). Ein weiterer Vorteil einer nur katalytischen Menge an sekundärem Amin ist der freizügige Einsatz von Ausgangsmaterialien, die einen Aminaustausch eingehen, wie dies beispielsweise bei Estern zu beobachten ist.

Die nur katalytische Menge an sekundärem Amin bei Aminomethylenierungen mit Amidacetalen ist auch ein Vorteil gegenüber Umsetzungen von Carbonsäuremethylestern mit Alkoxy-bis-(dialkylamino)-methanen (Aminalestern) oder Tris-(dialkylamino)-methanen, wo durch Freisetzung von 1 bzw. 2 Äquivalenten an Amin in stärkerem Maße eine Amidbildung stattfinden kann.

Die unerwünschte Amidbildung kann bei gleichbleibender Ausbeute deutlich gesenkt werden; hierzu wird auf das Ausführungsbeispiel 2 hingewiesen.

Die genaue Rolle des Eingreifens von sekundärem Amin in die chemische Reaktion des erfindungsgemäßen Verfahrens ist nicht mit Sicherheit bekannt, steht aber in Übereinstimmung mit der Vorstellung, daß das Formamidacetal teilweise diisoziiert vorliegt. In Gegenwart des sekundären Amins kann Alkohol-/Aminaustausch unter Bildung eines Aminalesters stattfinden, der möglicherweise das eigentlich kondensierende (aminomethylenierende) Agens darstellt. Bei dieser Kondensation wird das sekundäre Amin abgespalten und steht sodann erneut zur Verfügung, so daß es nur in katalytischen Mengen gegenwärtig zu sein braucht.

Es ist dem Fachmann bekannt, daß es neben den oben genannten Orthoamiden noch andere Verbindungen gibt, die unter den Reaktionsbedingungen der Aminomethylenierung sekundäres Amin freisetzen können. Diese sind ebenfalls in den genannten katalytischen Mengen wirksam. Solche anderen Verbindungen sind beispielsweise die Salze schwacher Säuren der sekundären Amine der Formel (IV), z.B. die Carbonate, Acetate, Propionate usw., ferner die den Carbonaten entsprechenden Carbamate und die CO₂-Addukte von sekundären Aminen.

### Beispiele

### Beispiel 1

60,7 g 98%iges DMF-Dimethylacetal, 31 g Acetonitril und 36,5 g DMF wurden in einem 0,3 1-V4A-Autoklaven zur Reaktion gebracht. Dimethylamin wurde über ein Schauglas zudosiert. Die Ausbeutebestimmung geschah durch GC mit internem Standard.

| Reaktionsbedingungen | Dimethylamin-Anteil (in %, bezogen auf DMF-Acetal-Einsatz) | Ausbeute (in % d.Th.) |
|---|---|---|
| 12 h 130°C | / | 7,2 |
| 24 h 130°C | / | 15,8 |
| 12 h 130°C | 25 | 88,7 |
| 12 h 130°C | 10 | 89,3 |
| 12 h 130°C | 3,5 | 60,6 |

### Beispiel 2

60,7 g 98%iges DMF-Acetal, 88 g Essigsäureethylester und 36,5 g DMF wurden analog Beispiel 1 umgesetzt. Man erhielt ein Gemisch von β-Dimethylamino-acrylsäureethylester und dem entsprechenden Methylester. Zusätzlich wurde der Anteil an Dimethylacetamid (DMA) bestimmt. Die Menge an DMA wurde auf den überschüssigen Essigester-Anteil bezogen.

| Reaktionsbedingungen | Dimethylamin-Anteil | Ausbeute (in % d.Th.) | Dimethylacetamid |
|---|---|---|---|
| 15 h 140°C | / | 12,0 | / |
| 10 h 150°C | 100 % | 76,3 | 35 % |
| 15 h 140°C | 50 % | 82,0 | 16 % |
| 15 h 140°C | 25 % | 83,8 | 5 % |
| 15 h 140°C | 12,5 % | 83,1 | 2 % |

### Beispiel 3

60,7 g 98%iges DMF-Acetal, 68,5 g o-Nitrotoluol und 36,5 g DMF wurden analog umgesetzt.

| Reaktionsbedingungen | Dimethylamin-Anteil | Ausbeute (in % d.Th.) | Bemerkung |
|---|---|---|---|
| 20 h 120°C | / | 46,7 | / |
| 20 h 130°C | / | 54,7 | 10 % Überschuß DMF-Acetal |
| 20 h 130°C | 30 % | 85,3 | |
| 20 h 130°C | 15 % | 87,6 | / |
| 20 h 120°C | 15 % | 76,0 | / |

### Beispiel 4

In einem 1 l Vierhalskolben wurden 132,1 g (1 Mol) Dimethylmalonat in 500 ml Methanol vorgelegt und anschließend bei Raumtemperatur 9 g (0,2 mol) Dimethylamin eingeleitet. Hierzu gab man innerhalb von 10 min 119,2 g (1 Mol) DMF-Dimethylacetal und heizte auf 50°C auf. Nach 1 h wurde eine Probe gezogen und mit Hilfe der Gaschromatographie analysiert. Der Umsatz zu Dimethylaminomethylen-malonsäuredimethylester betrug 89 %. In einem Vergleichsversuch ohne Dimethylaminzusatz wurde nach 1 h nur ein Umsatz von 66 % beobachtet.

### Beispiel 5

Anstelle von Methanol wurde im Beispiel 5 die entsprechende Menge DMF eingesetzt. Der Umsatz nach 1 h lag bei 95 %.

### Beispiel 6

24 g DMF-Dimethylacetal, 17,4 g Aceton, 14,6 g DMF und 2,3 g Dimethylamin wurden in einem V4A-Autoklaven 12 Stunden auf 100°C erhitzt. Nach Einengen im Wasserstrahlvakuum destillierte man den Rückstand bei 1 mbar über eine 30 cm lange Füllkörperkolonne. 4-Dimethylamino-3-buten-2-on wurde in 76,8 % der Theorie als 99,6 %ige Ware erhalten.

## Patentansprüche

1. Verfahren zur Herstellung von Aminomethylenverbindungen der Formel 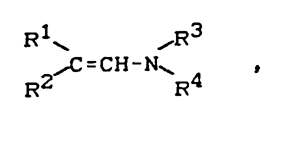 in der
R¹ Nitro, Cyano, Isonitrilo, COR⁹, CSR⁹, COOR⁹, COSR⁹, CON(R⁹,R¹⁰), SO₂R⁹, SO₃R⁹, CONH₂ oder einen an der Position des bevorzugten nukleophilen Angriffs elektronenarmen Aromaten oder Heteroatomaten darstellt,
R² unabhängig von R¹ den Bedeutungsumfang von R¹ annehmen kann und außerdem Wasserstoff, OR⁹, SR⁹, N(R⁹,R¹⁰), geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatom 1 oder 2 aus der Gruppe von NOS sind, darstellt,
R³ und R⁴ unabhängig voneinander geradkettiges oder verzweigtes C₁-C₈-Alkyl, C₂-C₈-Alkenyl, C₂-C₈-Alkoxyalkyl oder C₃-C₈-Alkoxyalkenyl darstellen und
R⁹ und R¹⁰ unabhängig voneinander geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cyclolkyl, C₆-C₁₂-Aryl, C₇-C₁₀-Aralkyl oder einen 5- bis 8-gliedrigen gesättigten oder ungesättigten heterocyclischen Ring, dessen Heteroatom 1 oder 2 aus der Gruppe von N, O, S sind, darstellen, wobei weiterhin R⁹ und R¹⁰ gemeinsam mit dem N-Atom, das sie substituieren, einen 5- bis 8-gliedrigen Ring bilden können, der ein weiteres Heteroatom aus der Gruppe von N, O, S enthalten kann,
dadurch gekennzeichnet, daß man C-H-acide Verbindungen der Formel 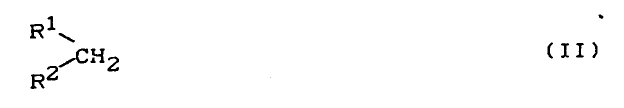 mit Formamidacetalen der Formel 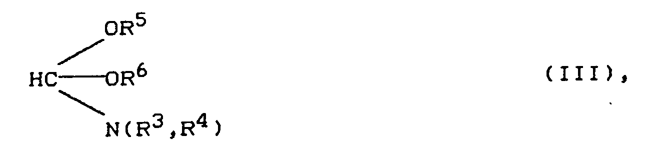 in denen
R¹ bis R⁴ den genannten Bedeutungsumfang haben und
R⁵ und R⁶ unabhängig von R³ und R⁴ und unabhängig voneinander den für R³ und R⁴ gegebenen Bedeutungsumfang annehmen,
in Gegenwart eines sekundären Amins der Formel 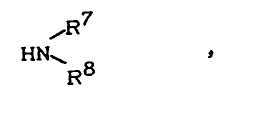 in der
R⁷ und R⁸ unabhängig von R³ und R⁴ und unabhängig voneinander den Bedeutungsumfang von R³ und R⁴ annehmen,
oder in Gegenwart von Substraten, die solche Amine unter den Reaktionsbedingungen freisetzen, bei 0 bis 200°C mit oder ohne Lösungsmittel umsetzt, wobei das molare Verhältnis von C-H-acider Verbindung zu Formamidacetal 1:3 bis 10:1 beträgt und die Menge an sekundärem Amin 1 bis 500 Mol-%, bezogen auf das Formamidacetal, beträgt.

2. Verfahren nach Anspruch 1, dadurch gekennzeichnet, daß die Umsetzung bei 20 bis 170°C durchgeführt wird.

3. Verfahren nach Anspruch 1, dadurch gekennzeichnet, daß das molare Verhältnis von C-H-acider Verbindung zu Formamidacetal 1:2 bis 5:1 beträgt.

4. Verfahren nach Anspruch 1, dadurch gekennzeichnet, daß die Menge an sekundärem Amin 2 bis 300 Mol-%, bezogen auf Formamidacetal, beträgt.

5. Verfahren nach Anspruch 4, dadurch gekennzeichnet, daß die Menge an sekundärem Amin 2 bis 25 Mol-%, bezogen auf Formamidacetal, beträgt.

6. Verfahren nach Anspruch 1, dadurch gekennzeichnet, daß bei Verwendung eines Losungsmittels ein solches aus der Gruppe Alkohole, Kohlenwasserstoffe, halogenierten Kohlenwasserstoffe, tertiären Amine, Ketone, Amide, Nitrile, Ether, Ester, Phosphorsäureamide und -ester, Sulfone, Sulfolane, N-Alkyllactame, Carbonate und Harnstoffderivate oder ein Gemisch mehrerer von ihnen eingesetzt wird.

7. Verfahren nach Anspruch 6, dadurch gekennzeichnet, daß ein Lösungsmittel aus der Gruppe der Alkohole, Ketone, Nitrile, Ester, Carbonate, Carboxamide, N-Alkyllactame, Tetraalkylharnstoffe, Dialkylsulfoxide, Dialkylsulfone und Ether eingesetzt wird.

## Claims

1. Process for preparing aminomethylene compounds of the formula 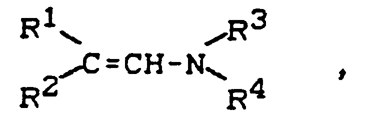 in which
R¹ represents nitro, cyano, isonitrilo, COR⁹, CSR⁹, COOR⁹, COSR⁹, CON(R⁹,R¹⁰), SO₂R⁹, SO₃R⁹ or CONH₂, or an aromatic or heteroaromatic residue which is electron-deficient at the site of preferred nucleophilic attack,
R² can assume the range of meaning of R¹, independently of R¹, and additionally represents hydrogen, OR⁹, SR⁹, N(R⁹,R¹⁰), straight-chain or branched C₁-C₈-alkyl, C₂-C₈-alkenyl, C₂-C₈-alkoxyalkyl, C₃-C₈-alkoxyalkenyl, C₃-C₈-cycloalkyl, C₆-C₁₂-aryl or C₇-C₁₀-aralkyl, or a 5- to 8-membered, saturated or unsaturated, heterocyclic ring whose 1 or 2 heteroatoms are selected from the group comprising N, O and S,
R³ and R⁴, independently of each other, represent straight-chain or branched C₁-C₈-alkyl, C₂-C₈-alkenyl, C₂-C₈-alkoxyalkyl or C₃-C₈-alkoxyalkenyl, and
R⁹ and R¹⁰, independently of each other, represent straight-chain or branched C₁-C₈-alkyl, C₂-C₈-alkenyl, C₂-C₈-alkoxyalkyl, C₃-C₈-alkoxyalkenyl, C₃-C₈-cycloalkyl, C₆-C₁₂-aryl, or C₇-C₁₀-aralkyl, or a 5- to 8-membered, saturated or unsaturated, heterocyclic ring whose 1 or 2 heteroatoms are selected from the group comprising N, O and S, where, furthermore, R⁹ and R¹⁰, together with the N atom which they substitute, can form a 5- to 8-membered ring which can contain a further heteroatom selected from the group comprising N, O and S,
which is characterized in that CH-acidic compounds of the formula 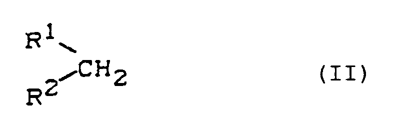 are reacted with formamide acetals of the formula 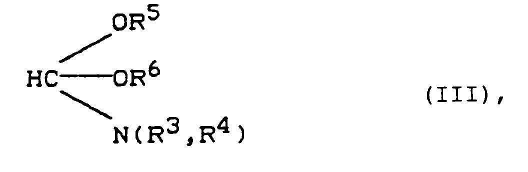 in which
R¹ to R⁴ have the said range of meaning and
R⁵ and R⁶, independently of R³ and R⁴ and independently of each other, assume the range of meaning stated for R³ and R⁴,
in the presence of a secondary amine of the formula 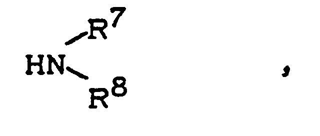 in which
R⁷ and R⁸, independently of R³ and R⁴ and independently of each other, assume the range of meaning of R³ and R⁴,
or in the presence of substrates which liberate such amines under the reaction conditions, at 0 to 200EC with or without solvent, the molar ratio of C-H-acidic compound to formamide acetal being 1:3 to 10:1 and the quantity of secondary amine being 1 to 500 mol %, based on the formamide acetal.

2. Process according to Claim 1, characterized in that the reaction is carried out at 20 to 170EC.

3. Process according to Claim 1, characterized in that the molar ratio of C-H-acidic compound to formamide acetal is 1:2 to 5:1.

4. Process according to Claim 1, characterized in that the quantity of secondary amine is 2 to 300 mol %, based on formamide acetal.

5. Process according to Claim 4, characterized in that the quantity of secondary amine is 2 to 25 mol %, based on formamide acetal.

6. Process according to Claim 1, characterized in that, when a solvent is used, either one selected from the group comprising alcohols, hydrocarbons, halogenated hydrocarbons, tertiary amines, ketones, amides, nitriles, ethers, esters, phosphoric acid amides and phosphoric acid esters, sulphones, sulpholanes, N-alkyllactams, carbonates and urea derivatives, or else a mixture of a number of these, is employed.

7. Process according to Claim 6, characterized in that a solvent is employed which is selected from the group comprising the alcohols, ketones, nitriles, esters, carbonates, carboxamides, N-alkyllactams, tetraalkylureas, dialkyl sulphoxides, dialkyl sulphones and ethers.

## Revendications

1. Procédé pour la préparation de composés aminométhyléniques de la formule 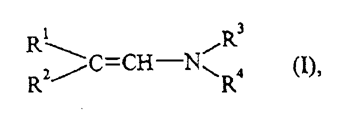 dans laquelle
R¹ représente un groupe nitro, cyano, isonitrilo, COR⁹, CSR⁹, COOR⁹, CORS⁹, CON(R⁹,R¹⁰), SO₂R⁹, SO₃R⁹, CONH₂ ou un composé aromatique ou hétéroaromatique pauvre en électrons à la position de l'attaque nucléophile préférée,
R² peut avoir la signification de R¹ indépendamment de R¹ et représente en outre un atome d'hydrogène, OR⁹, SR⁹, N(R⁹, R¹⁰), un groupe alkyle en C₁-C₈, alcényle en C₂-C₈, alcoxyalkyle en C₂-C₈, alcoxyalcényle en C₃-C₈, cycloalkyle en C₃-C₈, aryle en C₆-C₁₂, aralkyle en C₇-C₁₀ linéaire ou ramifié ou un noyau hétérocyclique saturé ou insaturé à de 5 à 8 éléments, dont l'hétéroatome 1 ou 2 est choisi parmi N, O, S,
R³ et R⁴ représentent indépendamment l'un de l'autre un groupe alkyle en C₁-C₈, alcényle en C₂-C₈, alcoxyalkyle en C₂-C₈ ou alcoxyalcényle en C₃-C₈ linéaire ou ramifié et
R⁹ et R¹⁰ représentent indépendamment l'un de l'autre un groupe alkyle en C₁-C₈, alcényle en C₂-C₈, alcoxyalkyle en C₂-C₈, alcoxyalcényle en C₃-C₈, cycloalkyle en C₃-C₈, aryle en C₆-C₁₂, aralkyle en C₇-C₁₀ linéaire ou ramifié ou un noyau hétérocyclique saturé ou insaturé à de 5 à 8 éléments, dont les hétéroatomes 1 ou 2 sont choisis parmi N, O, S, R⁹ et R¹⁰ pouvant en outre former ensemble avec l'atome N qu'ils substituent un noyau à de 5 à 8 éléments qui peut contenir un autre hétéroatome choisi parmi N, O, S,
caractérisé en ce que l'on fait réagir à de 0 à 200°C avec ou sans solvant des composés acides C-H de la formule 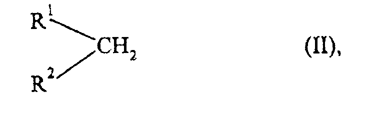 avec des formamidacétals de la formule 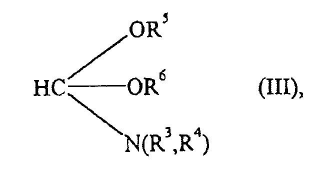 dans laquelle
R¹ à R⁴ ont la signification citée et
R⁵ et R⁶ ont, indépendamment de R³ et R⁴ et indépendamment l'un de l'autre, la signification indiquée pour R³ et R⁴,
en présence d'une amine secondaire de la formule 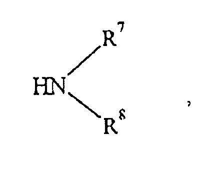 dans laquelle
R⁷ et R⁸ ont indépendamment de R³ et R⁴ et indépendamment l'un de l'autre la signification de R³ et R⁴,
ou en présence de substrats qui libèrent de telles amines dans les conditions de la réaction, le rapport molaire du composé acide C-H au formamidacétal étant de 1:3 à 10:1 et la quantité en amine secondaire étant de 1 à 500% en mol, rapportés au formamidacétal.

2. Procédé selon la revendication 1, caractérisé en ce que la réaction est réalisée à de 20 à 170°C.

3. Procédé selon la revendication 1, caractérisé en ce que le rapport molaire du composé acide C-H au formamidacétal est de 1:2 à 5:1.

4. Procédé selon la revendication 1, caractérisé en ce que la quantité d'amine secondaire est de 2 à 300% en mol, rapportée au formamidacétal.

5. Procédé selon la revendication 4, caractérisé en ce que la quantité d'amine secondaire est de 2 à 300% en mol, rapportée au formamidacétal.

6. Procédé selon la revendication 1, caractérisé en ce que lors de l'utilisation d'un solvant on utilise un solvant choisi parmi les alcools, les hydrocarbures, les hydrocarbures halogénés, les amines tertiaires, les cétones, les amides, les nitriles, les éthers, les esters, les amides et les esters d'acide phosphorique, les sulfones, les sulfolanes, les N-alkyl-lactames, les carbonates et les dérivés de l'urée ou un mélange de plusieurs d'entre eux.

7. Procédé selon la revendication 6, caractérisé en ce que l'on utilise un solvant choisi parmi les alcools, les cétones, les nitriles, les esters, les carbonates, les carboxamides, les N-alkylactames, les tétralkylurées, les dialkylsulfoxydes, les dialkylsulfones et les éthers.
